# EUROPEAN PATENT APPLICATION

(11) **EP 2 443 987 A1**
(43) Date of publication of application: **25.04.2012**
(21) Application number: 11185774.4
(22) Date of filing: 19.10.2011
(51) Int. Cl.: A61B 1/12, A61B 1/00, A61B 1/005, A61B 1/313

(54) **Endoscope and endoscope system**

(30) Priority: 20.10.2010 JP 2010235683
(71) Applicant: Fujifilm Corporation, Minato-ku Tokyo 106-8620 (JP)
(72) Inventor: Yoshida, Koji, Ashigarakami-gun, Kanagawa, 258-8538 (JP); Shibuya, Hiroshi, Ashigarakami-gun, Kanagawa, 258-8538 (JP)
(74) Representative: Klunker . Schmitt-Nilson . Hirsch

(57) **Abstract**

Provided is an endoscope (10) including: a hand operation section (12); an insertion section (14) including a leading end part (42), a curving part (40), and a flexible part (38) that are placed in the stated order from a leading end side to a base end side on which the hand operation section (12) is placed, the leading end part (42) having a leading end surface (44) on which an observation window for observing an inside of a subject's body is provided, the curving part (40) being remotely operated for curving by operating the hand operation section (12) so as to cause the leading end part (42) to face a desired direction, the flexible part (38) connecting the curving part (40) with the hand operation section (12) and being able to curve in a desired direction along an insertion into the subject's body; a gas supply channel formed inside of the insertion section (14); and a gas jetting port (54, 64, 68) that is communicated with the gas supply channel, and is placed so as to be closer to the base end side than the leading end surface (44) on which the observation window (46) is provided. Accordingly, it is possible to prevent bubbles generated near the gas jetting port (54, 64, 68) from blocking the field of view of the observation window (46) when a constant-pressure gas is supplied via an opening formed on a leading end surface (44) of a flexible endoscope (10), to thereby secure the field of view.

## Description

### BACKGROUND OF THE INVENTION

### Field of the Invention

The present invention relates to an endoscope and an endoscope system, and more particularly, to an endoscope and an endoscope system in which a constant-pressure gas is supplied from a gas supply apparatus into a lumen of a subject via an opening provided in a leading end part of a flexible endoscope inserted into the lumen, to thereby perform observation and treatment of the inside of the lumen.

### Description of the Related Art

Up to now, in medical fields, a medical diagnosis utilizing an endoscope is widely performed. In particular, an image-pickup element such as a CCD is built in an insertion leading end part of the endoscope inserted into a body cavity, and an image inside of the body cavity is photographed by the built-in image-pickup element. The image is subjected to signal processing by a processor apparatus to be displayed on a monitor. A doctor observes for a diagnosis the processed image. Otherwise, a treatment tool is inserted from a channel for treatment tool insertion, to thereby perform treatment such as collection of a sample and excision of a polyp.

In addition, in a laparoscope-assisted surgery in which curative treatment is performed without opening an abdomen in order to realize a less-invasive operation for a patient, a rigid endoscope for observation and a treatment tool for the curative treatment are guided into a body cavity via a plurality of trocars inserted into the abdomen of the patient. At this time, an insufflation apparatus that supplies an insufflation gas into an abdominal cavity is used to secure the field of view of the rigid endoscope and secure a region for operating the treatment tool.

In addition, for example, a flexible insertion section of a flexible endoscope is inserted into a lumen of a stomach, a large intestine, or other organs, and diagnosis and treatment are performed on the inside of the lumen. In this case, a treatment tool is inserted into the lumen via a forceps channel (treatment tool channel) of the flexible endoscope, to thereby perform the curative treatment. Similarly at this time, a constant-pressure supplied gas such as carbon dioxide gas is supplied into the lumen.

For example, WO 2007/080971 discloses that an endoscope is inserted into a stomach through an over tube that guides the insertion into a body, a tube connected to a gas supply apparatus is connected to a gas supply/water supply channel through a universal cable of the endoscope, and a gas is supplied into the stomach through the tube from the gas supply apparatus.

In addition, Japanese Patent Application Laid-Open No. 2007-296164 discloses that a gas for securing the field of view of an observation window and an insufflation gas are jetted from an injection nozzle provided in a leading end hard part of an endoscope insertion section.

In addition, for example, Japanese Patent Application Laid-Open No. 2009-106360 discloses a laparoscope-assisted surgery system including two endoscopes, that is, a rigid endoscope and a flexible endoscope, in which: a plurality of trocars are inserted into an abdomen of a patient; the rigid endoscope is inserted into an abdominal cavity from one of the trocars; an insufflation gas supplied from an insufflation apparatus is guided into the abdominal cavity via the other trocars; and the flexible endoscope is further inserted into a lumen of a large intestine or other organs. Then, in this system, carbon dioxide gas, which is adjusted to a predetermined pressure, is guided into the abdominal cavity via the other trocars and is also supplied into the lumen via a treatment tool channel of the flexible endoscope, as the insufflation gas from the insufflation apparatus.

In addition, Japanese Patent Application Laid-Open No. 2005-279060 discloses that a flexible endoscope includes an insufflation guide pipe for performing insufflation by supplying a gas into an abdominal cavity of a patient, and performs endoscopic examination on the inside of a lumen of a large intestine or other organs of the patient, and in this flexible endoscope, carbon dioxide gas supplied from a gas supply apparatus can be supplied from a leading end part of an insertion section via a gas supply pipe conduit in a universal code.

Unfortunately, for example, when a constant-pressure gas is supplied using an over tube including: a gas supply injection port formed in a leading end part of an endoscope, such as a forceps port of a flexible endoscope; and a gas supply/water supply channel on a leading end surface of an insertion section, if the leading end part of the endoscope is submerged under water or if a bodily fluid or water is attached to the leading end surface of the endoscope, bubbles are generated near a gas jetting port, and the bubbles are attached to an observation window (image-pickup apparatus) formed on the leading end surface, so that the field of view is blocked unfavorably.

### SUMMARY OF THE INVENTION

The present invention has been made in view of the above-mentioned circumstances, and therefore has an object to provide an endoscope and an endoscope system that can prevent bubbles generated near a gas jetting port from blocking the field of view of an observation window when a constant-pressure gas is supplied via an opening formed on a leading end surface of a flexible endoscope, to thereby secure the field of view.

In order to achieve the above-mentioned object, a first aspect of the present invention provides an endoscope including: an endoscope comprising:
a hand operation section;
an insertion section including a leading end part, a curving part, and a flexible part that are placed in the stated order from a leading end side to a base end side on which the hand operation section is placed,
   the leading end part having a leading end surface on which an observation window for observing an inside of a subject's body is provided,
   the curving part being remotely operated for curving by operating the hand operation section so as to cause the leading end part to face a desired direction,
   the flexible part connecting the curving part with the hand operation section and being able to curve in a desired direction along an insertion into the subject's body;
a gas supply channel formed inside of the insertion section; and
a gas jetting port that is communicated with the gas supply channel, and is placed so as to be closer to the base end side than the leading end surface on which the observation window is provided.

As described above, because the gas jetting port is placed so as to be closer to the base end side than the leading end surface on which the observation window is provided, it is possible to prevent bubbles generated by the gas jetted from the gas jetting port from blocking the field of view of the observation window, and hence the field of view of the observation window can be secured.

In addition, according to a second aspect of the present invention, the gas jetting port is placed on a stepped surface that is formed by providing a step on part of the leading end surface so as to be lower toward the base end than the leading end surface.

As described above, because the gas jetting port is placed on the stepped surface that is lower by one step toward the base end than the leading end surface, the gas jetting port and the observation window can be spaced apart from each other. As a result, it is possible to prevent bubbles generated by the gas jetted from the gas jetting port from blocking the field of view of the observation window, and hence the field of view of the observation window can be secured.

In addition, according to a third aspect of the present invention, the gas jetting port is formed on a side surface of the leading end part.

As described above, because the gas jetting port is formed on the side surface of the leading end part, the gas jetting port and the observation window can be spaced apart from each other. As a result, it is possible to prevent bubbles generated by the gas jetted from the gas jetting port from blocking the field of view of the observation window, and hence the field of view of the observation window can be secured.

In addition, according to a fourth aspect of the present invention, the gas jetting port is formed so as to be closer to the base end side than the curving part.

As described above, because the gas jetting port is formed so as to be closer to the base end than the curving part, the gas jetting port can be spaced sufficiently apart from the leading end part. Even if the leading end part is submerged under water inside of a stomach or a large intestine, the gas jetting port is prevented from being submerged under water. Accordingly, it is possible to suppress generation of bubbles from being generated by the gas jetted from the gas jetting port.

In addition, according to a fifth aspect of the present invention, the gas jetting port is formed on a side surface of the flexible part.

As described above, because the gas jetting port is formed on the side surface of the flexible part, the gas jetting port can be spaced sufficiently apart from the leading end part. Even if the leading end part is submerged under water inside of a stomach or a large intestine, the gas jetting port is prevented from being submerged under water. Accordingly, it is possible to suppress bubbles from being generated by the gas jetted from the gas jetting port.

In addition, according to a sixth aspect of the present invention, a plurality of the gas jetting ports are formed in a circumferential direction of the flexible part on the side surface of the flexible part.

In addition, according to a seventh aspect of the present invention, a plurality of the gas jetting ports are formed in an axial direction of the flexible part on the side surface of the flexible part.

As described above, because the plurality of gas jetting ports are formed at positions different in the circumferential direction or the axial direction of the flexible part, even if any of the gas jetting ports is blocked by an inner wall of a lumen or the like, the gas can still be jetted from the other gas jetting ports.

In addition, according to an eighth aspect of the present invention, a pipe conduit portion of the gas supply channel communicated with the gas jetting port is smoothly curved in proximity to the gas jetting port.

This can improve cleaning performance when the gas supply channel and the pipe conduit portion thereof communicated with the gas jetting port are cleaned with a brush or other tools.

In addition, according to a ninth aspect of the present invention, a pipe conduit portion of the gas supply channel communicated with the gas jetting port is bent at one of a substantially right angle and an acute angle to an axial direction of the endoscope in proximity to the gas jetting port.

As described above, because the pipe conduit portion communicated with the gas jetting port is bent at a right or acute angle to the axial direction of the endoscope, the gas can be jetted from the gas jetting port in a direction farther from the observation window. Accordingly, it is possible to prevent generated bubbles from approaching the observation window, and the field of view of the observation window can be sufficiently secured.

Similarly, in order to achieve the above-mentioned object, a tenth aspect of the present invention provides an endoscope system including: the endoscope according to any one of the first to ninth aspects; and a gas supply apparatus that supplies a gas to the gas supply channel of the endoscope.

As described above, because the gas is supplied from the gas supply apparatus to the endoscope in which the gas jetting port is spaced apart from the leading end surface on which the observation window is provided, even if bubbles are generated by the gas near the gas jetting port, the bubbles do not block the field of view of the observation window, and hence the field of view of the observation window can be sufficiently secured.

In addition, according to an eleventh aspect of the present invention, the gas supply apparatus continuously supplies the gas.

This enables continuous insufflation of a lumen, and hence observation and treatment using the endoscope can be properly performed.

As described above, according to the present invention, because the gas jetting port is placed so as to be closer to the base end side than the leading end surface on which the observation window is provided, it is possible to prevent bubbles generated by the gas jetted from the gas jetting port from blocking the field of view of the observation window, and hence the field of view of the observation window can be secured.

### BRIEF DESCRIPTION OF THE DRAWINGS

Fig. 1 is an external view schematically illustrating an overall configuration of a first embodiment of an endoscope system according to the present invention;
Fig. 2 is a perspective view illustrating a leading end part of an insertion section;
Fig. 3 is a plan view illustrating a leading end surface;
Fig. 4 is a cross-sectional view taken along the dashed-dotted line 4A-4A in Fig. 3;
Fig. 5 is a plan view illustrating a leading end surface of an endoscope according to a second embodiment of the present invention;
Fig. 6 is a cross-sectional view taken along the dashed-dotted line 6A-6A in Fig. 5;
Fig. 7 is a perspective view illustrating an insertion section of an endoscope according to a third embodiment of the present invention; and
Fig. 8 is a perspective view illustrating an insertion section of an endoscope according to a modified example of the third embodiment.

### DETAILED DESCRIPTION OF THE PREFERRED EMBODIMENTS

Hereinafter, an endoscope and an endoscope system according to the present invention are described in detail with reference to the attached drawings.

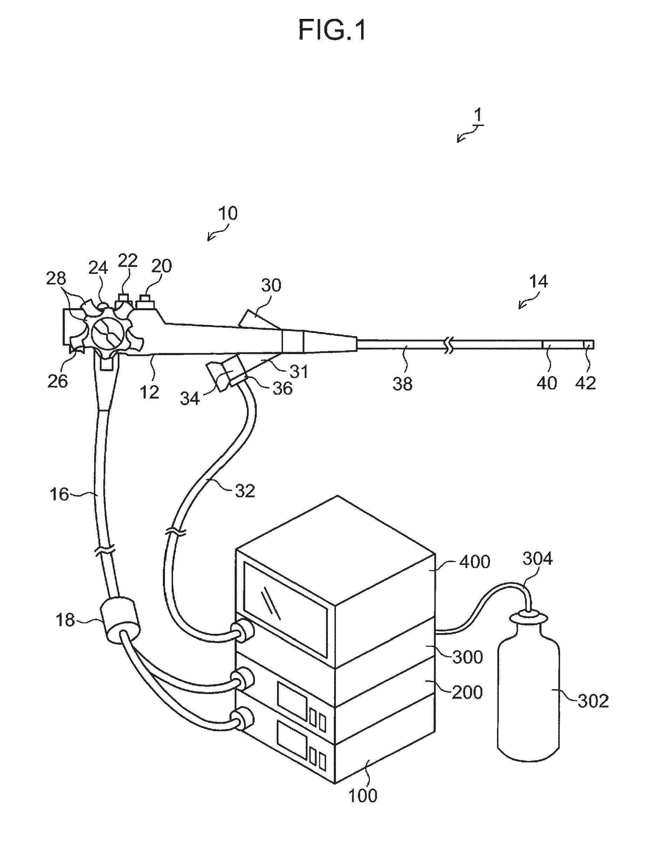

Fig. 1 is an external view schematically illustrating an overall configuration of a first embodiment of an endoscope system according to the present invention.

As illustrated in Fig. 1, an endoscope system 1 mainly includes an endoscope (flexible endoscope) 10, a light source apparatus 100, an endoscope processor 200, a gas supply apparatus 300, and a monitor apparatus 400. These apparatuses do not necessarily need to be configured as separate members as illustrated in Fig. 1. For example, the light source apparatus 100 may be built in the endoscope processor 200.

The endoscope 10 includes a hand operation section 12 and an insertion section 14 provided continuously with the hand operation section 12. An operator grips the hand operation section 12 placed on the base end side to operate the endoscope 10, and inserts the leading end of the insertion section 14 into a lumen of a stomach, a large intestine, or other organs of a subject, to thereby perform observation and diagnosis or curative treatment.

A universal cable 16 is connected to the hand operation section 12, and an LG connector 18 is provided to the leading end of the universal cable 16. The LG connector 18 is detachably coupled to the light source apparatus 100, whereby illumination light is supplied to an illumination optical system (not illustrated) provided in a leading end part of the insertion section 14. In addition, an electrical connector is connected to the LG connector 18 via the universal cable 16, and the electrical connector is detachably coupled to the endoscope processor 200. With this configuration, data of an observation image obtained by the endoscope 10 is outputted to the endoscope processor 200, and the observation image is displayed on the monitor apparatus 400 connected to the endoscope processor 200.

In addition, the hand operation section 12 is provided with a gas supply/water supply button 20, a suction button 22, a shutter button 24, a seesaw switch 26 for a zoom operation, a pair of angle knobs 28, and forceps insertion parts 30 and 31.

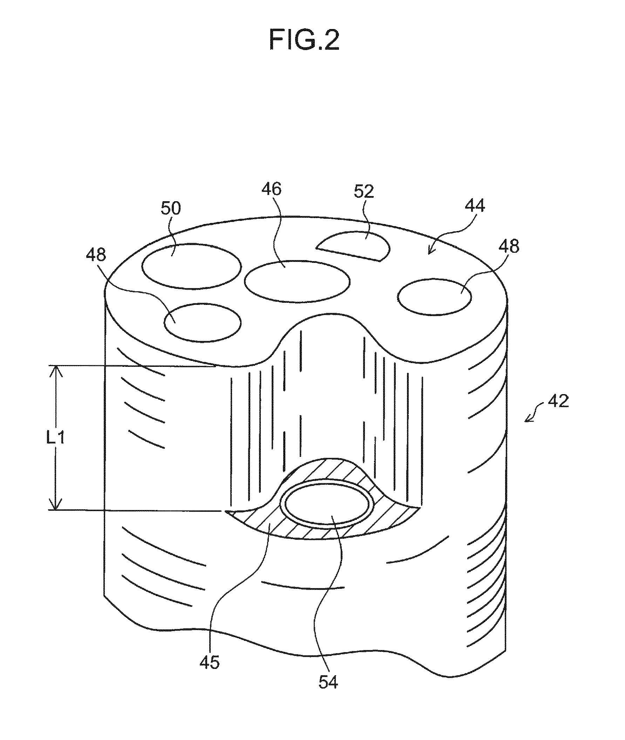

The forceps insertion part 30 is communicated with a forceps port for treatment (see Fig. 2) in the endoscope leading end part via a forceps channel (not illustrated) formed inside of the insertion section 14. In addition, the other forceps insertion part 31 is communicated with a forceps port for gas supply (see Fig. 2) in the endoscope leading end part via a gas supply channel (not illustrated) formed inside of the insertion section 14. An insertion port adapter 34 is provided to the forceps insertion part 31, and serves to supply carbon dioxide gas as a constant-pressure supplied gas into the lumen, and a gas supply tube 32 is coupled to a gas supply ferrule 36 of the insertion port adapter 34. In addition, another end of the gas supply tube 32 is coupled to the gas supply apparatus 300.

In addition, a carbon dioxide gas cylinder 302 is coupled to the gas supply apparatus 300 via a high-pressure gas tube 304. The carbon dioxide gas is reserved in the carbon dioxide gas cylinder 302 in the form of a liquid. Then, the gas supply apparatus 300 feeds the carbon dioxide gas reserved in the carbon dioxide gas cylinder 302 as the constant-pressure gas adjusted to a predetermined pressure into the gas supply channel from the forceps insertion part 31 via the gas supply tube 32, and then jets the carbon dioxide gas into the lumen of the subject from the forceps port for gas supply (see Fig. 2, to be described later) in the endoscope leading end part.

Note that, the gas supply by the gas supply apparatus 300 is continuously performed throughout the observation and treatment by the endoscope 10. Such continuous gas supply by the gas supply apparatus 300 enables continuous insufflation of the lumen.

In addition, in the insertion section 14, a leading end part 42, a curving part 40, and a flexible part 38 are placed in the stated order from the leading end side toward the base end side on which the hand operation section 12 is placed.

An observation window to be described later is formed on a leading end surface of the leading end part 42. The curving part 40 is remotely operated for curving by turning the pair of angle knobs 28 provided in the hand operation section 12. This enables the leading end part 42 to face a desired direction. In addition, the flexible part 38 connects the hand operation section 12 with the curving part 40, and is formed of a flexible member so as to be bendable in a desired direction along the insertion into a subject's body.

Fig. 2 is a perspective view illustrating the leading end part 42 of the insertion section 14.

As illustrated in Fig. 2, an observation window 46 on the inner side of which an image-pickup apparatus (CCD camera) is provided, illumination windows 48 and 48 for illuminating an observation range, a forceps port 50 for treatment, and a gas supply/water supply nozzle 52 are provided on a leading end surface 44 of the leading end part 42.

An optical system (observation optical system) for taking in image light inside of the subject's body is provided on the inner side of the observation window 46, and the taken-in image light representing an observation image is received by the CCD and is sent to the endoscope processor 200 via a signal cable. Then, the image light is converted into a video signal by the endoscope processor 200, and the observation image is displayed on the monitor apparatus 400 connected to the endoscope processor 200.

As illustrated in Fig. 2, the two illumination windows 48 and 48 are placed symmetrically with respect to the observation window 46, and an observation region inside of the subject's body is illuminated with the illumination light from the light source apparatus 100. The light from the light source apparatus 100 is guided to the illumination window 48 by an optical fiber (light guide) provided in the insertion section 14. Then, the illumination light is emitted through an illumination lens provided at the leading end and a cover glass fitted into the illumination window 48.

The forceps port 50 for treatment is connected to the forceps channel (not illustrated) provided inside of the insertion section 14, and is communicated with the forceps insertion part 30 of the operation part 12. The leading ends of forceps or other various treatment tools inserted through the forceps insertion part 30 are exposed at the forceps port 50 for treatment via the forceps channel.

The gas supply/water supply nozzle 52 serves to spray a cleaning solution and pressurized air when the observation window 46 becomes dirty, to thereby clean the observation window 46. The gas supply/water supply nozzle 52 jets fluids such as pressurized air and cleaning water toward the observation window 46 in response to a gas supply operation and a water supply operation on the gas supply/water supply button 20 provided in the operation part 12. As a result, a bodily fluid and dirt attached to the observation window 46 are wiped, to thereby secure an excellent field of view.

Further, in the present embodiment, a forceps port 54 for gas supply is placed on a stepped surface 45 that is formed by providing a step on part of the leading end surface 44 so as to be lower toward the base end than the leading end surface 44.

The forceps port 54 for gas supply is communicated with the forceps insertion part 31 via the gas supply channel. As described above (see Fig. 1), the insertion port adapter 34 is attached to the forceps insertion part 31, and the gas supply tube 32 coupled to the gas supply apparatus 300 is coupled to the gas supply ferrule 36 of the insertion port adapter 34. Then, the carbon dioxide gas is supplied into the lumen by the gas supply apparatus 300 via the gas supply tube 32 and the forceps port 54 for gas supply.

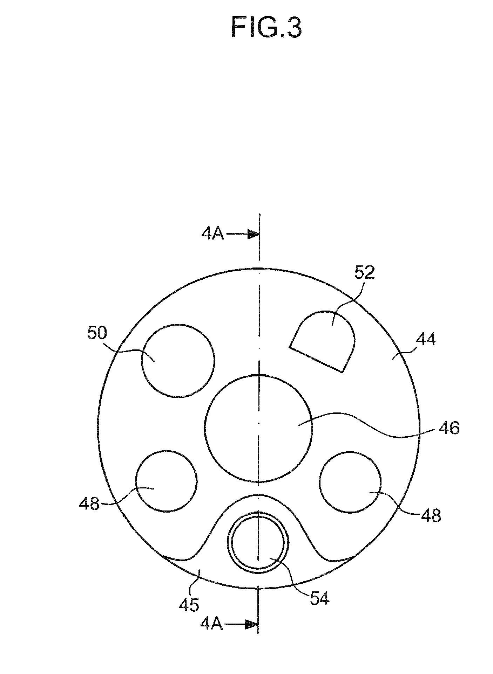

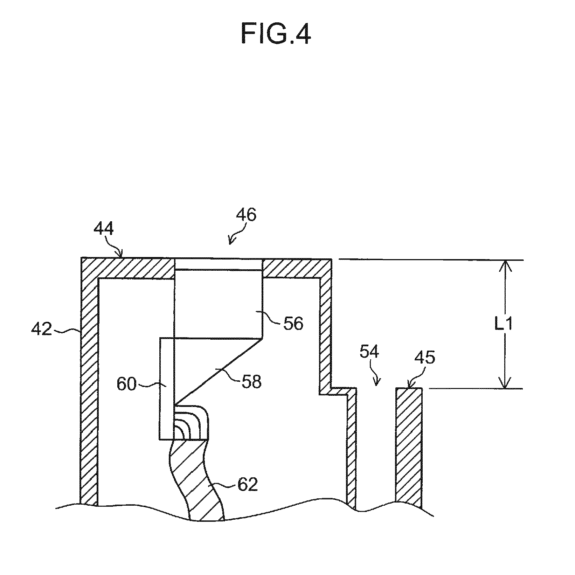

Next, Fig. 3 is a plan view illustrating the leading end surface 44, and Fig. 4 is a cross-sectional view taken along the dashed-dotted line 4A-4A in Fig. 3.

As illustrated in Fig. 3, the observation window 46 is placed at substantially the center of the leading end surface 44, the two illumination windows 48 and 48 are placed substantially symmetrically with respect to the observation window 46, and the forceps port 50 for treatment is placed next to the observation window 46. In addition, the gas supply/water supply nozzle 52 that jets, to the observation window 46, the cleaning water for cleaning the observation window 46 and the pressurized air for drying the same is placed so as to face the observation window 46.

As illustrated in Fig. 4, on the inner side of the observation window 46 are provided: a lens group 56 constituting the observation optical system; a prism 58 as an optical path changing device that bends by 90° visible light that has entered the lens group 56; and a CCD 60 as an image-pickup device that is located below the prism 58 and performs normal photographing with the visible light. The subject light bent by the prism 58 forms an image on a light receiving surface of the CCD 60.

Then, the subject light is converted into an electrical signal by the CCD 60, and the electrical signal is transmitted via a signal cable 62. The signal cable 62 is inserted through the insertion section 14, the hand operation section 12, the universal cable 16, and other members to be connected to the endoscope processor 200 via the electrical connector.

With this configuration, the observation image taken in through the observation window 46 is formed on the light receiving surface of the CCD 60 to be converted into an electrical signal, and the electrical signal is outputted to the endoscope processor 200 via the signal cable 62 to be converted into a video signal, whereby the observation image is displayed on the monitor apparatus 400 connected to the endoscope processor 200.

In addition, as illustrated in Fig. 4, the forceps port 54 for gas supply is formed on the stepped surface 45 that is lower by a difference in level L1 than the leading end surface 44, and the constant-pressure supplied gas (carbon dioxide gas) is jetted from the forceps port 54 for gas supply.

As described above, the forceps port 54 for gas supply is formed on the stepped surface 45 that is lower by one step toward the base end side than the leading end surface 44 on which the observation window 46 is formed. Accordingly, even if bubbles are generated by the gas jetted from the forceps port 54 for gas supply, the bubbles do not block the field of view of the observation window 46, and hence the field of view of the observation window 46 can be sufficiently secured.

Note that, the difference in level L1 can be designed to an arbitrary value in the leading end part 42 from the level of the leading end surface 44 to the boundary with the curving part 40. Meanwhile, if the value of L1 is excessively small and there is little difference in level with respect to the leading end surface 44, the bubbles generated near the forceps port 54 for gas supply may block the field of view of the observation window 46, and hence L1 needs to have a certain amount of value.

Next, a second embodiment of the present invention is described.

The overall configuration of an endoscope system of the second embodiment is similar to that of the first embodiment.

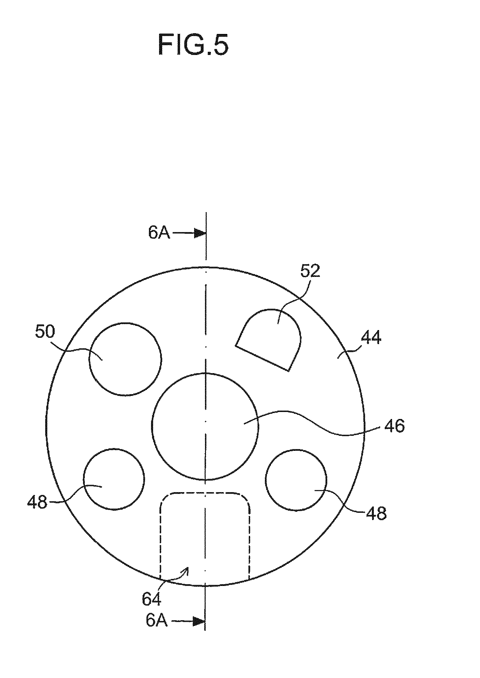

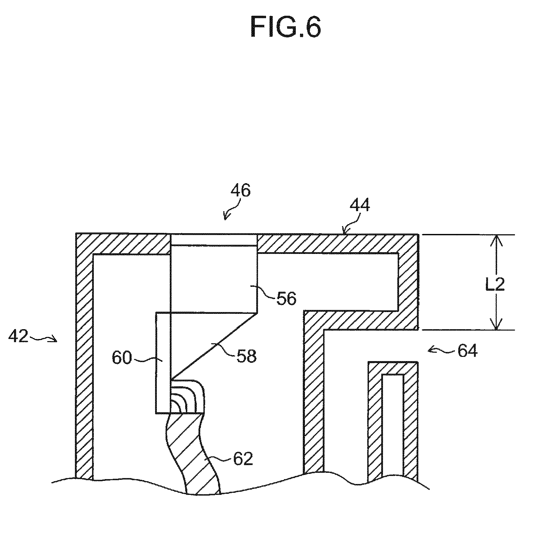

Fig. 5 is a plan view illustrating a leading end surface of an endoscope according to the second embodiment, and Fig. 6 is a cross-sectional view taken along the dashed-dotted line 6A-6A in Fig. 5.

Note that, the same constituent elements as those in the first embodiment are denoted by the same reference signs, and detailed description thereof is omitted.

As illustrated in Fig. 5 and Fig. 6, in the present embodiment, a forceps port 64 for gas supply is formed on a side surface of the leading end part 42.

In this way, the forceps port 64 for gas supply is formed on the side surface of the leading end part 42, and a gas jetting port is spaced apart from the observation window 46, whereby it is possible to prevent bubbles generated by the jetted gas from blocking the field of view of the observation window 46.

Note that, as illustrated in Fig. 6, on the side surface of the leading end part 42, the forceps port 64 for gas supply is formed at a position away by L2 toward the base end from the leading end surface 44. Similarly to the difference in level L1 in the first embodiment, L2 can be designed to an arbitrary value from the level of the leading end surface 44 to the boundary with the curving part 40.

In Fig. 6, an inner pipe conduit near the forceps port 64 for gas supply has a shape bent at a substantially right angle to the axial direction of the endoscope. Considering that the inside of the pipe conduit is cleaned with a brush or other tools, a smoothly curved shape is more preferable than the shape bent at a right angle in terms of an improvement in cleaning performance, but the gas may be jetted in a direction farther from the leading end surface 44 by adopting a shape bent at a right or acute angle.

In addition, the shape of an opening of the forceps port 64 for gas supply formed on the side surface of the leading end part 42 is not limited to a circle, and may be an ellipse, a shape like a track in an athletic field, which is obtained by combining a rectangle and semicircles, or a rectangle having rounded corners.

Next, a third embodiment of the present invention is described.

The overall configuration of an endoscope system of the third embodiment is also similar to that of the first embodiment.

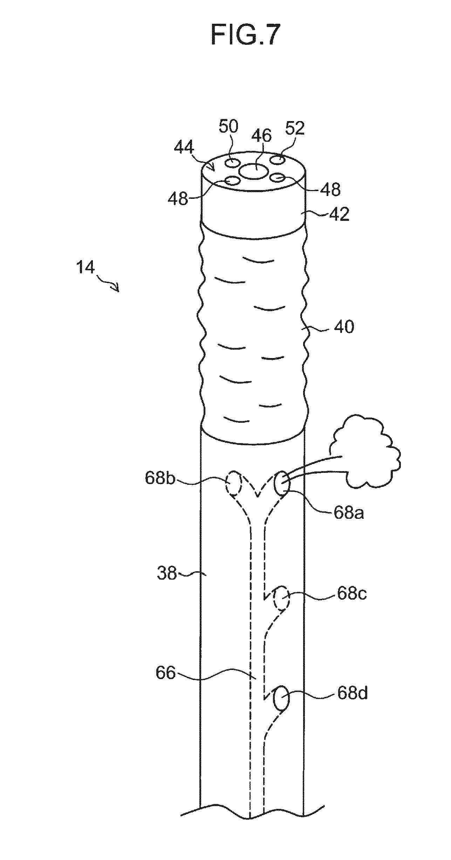

Fig. 7 is a perspective view illustrating an insertion section of an endoscope according to the present embodiment.

As illustrated in Fig. 7, the insertion section 14 includes the flexible part 38, the curving part 40, and the leading end part 42, and the observation window 46, the illumination windows 48, the forceps port 50 for treatment, and the gas supply/water supply nozzle 52 are provided on the leading end surface 44.

In addition, a gas supply channel 66 is formed inside of the flexible part 38, and a plurality of jetting ports 68 (68a, 68b, 68c, and 68d) that are communicated with the gas supply channel 66 and jet the gas are formed on a side surface of the flexible part 38.

In Fig. 7, the plurality of jetting ports 68 (68a, 68b, 68c, and 68d) may be provided, for example, at positions different in the circumferential direction like a positional relation between 68a and 68b or at positions different in the axial direction like a positional relation between 68c and 68d on the side surface of the flexible part 38.

In the case where the jetting port is provided in the leading end part 42, if the leading end part 42 is submerged under water inside of the lumen of the stomach, the large intestine, or other organs, bubbles are generated by the gas jetted from the jetting port submerged under water. In contrast, because the jetting ports 68 are provided on the side surface of the flexible part 38 in the present embodiment, even if the leading end part 42 of the endoscope is submerged under water inside of the lumen, the jetting ports 68 formed on the side surface of the flexible part 38 are not submerged under water, so that it is possible to prevent bubbles from being generated.

Note that, the reason why the number of the jetting ports 68 (68a, 68b, 68c, and 68d) formed on the side surface of the flexible part 38 is more than one is that when the flexible part 3 8 comes into contact with an inner wall of the lumen, even if any of the jetting ports 68 is blocked by the inner wall of the lumen, the gas can still be jetted from the other jetting ports 68.

In addition, because the jetting ports 68 and the gas supply channel 66 are provided in the flexible part 38, the jetting port and piping in the leading end part 42 can be reduced, and a packaging density of the leading end part 42 can be lower, leading to a reduction in diameter. As a result, the image-pickup module constitutes a large portion of the leading end part 42, and hence the reduction in piping is significantly effective.

In addition, the reason why the jetting ports 68 and the gas supply channel 66 are provided in not the curving part 40 but the flexible part 38 is that if the jetting ports 68 and the gas supply channel 66 are provided in the curving part 40, a deterioration in the curving part 40, whose strength is originally low, is accelerated by cleaning with a cleaning brush or other reasons.

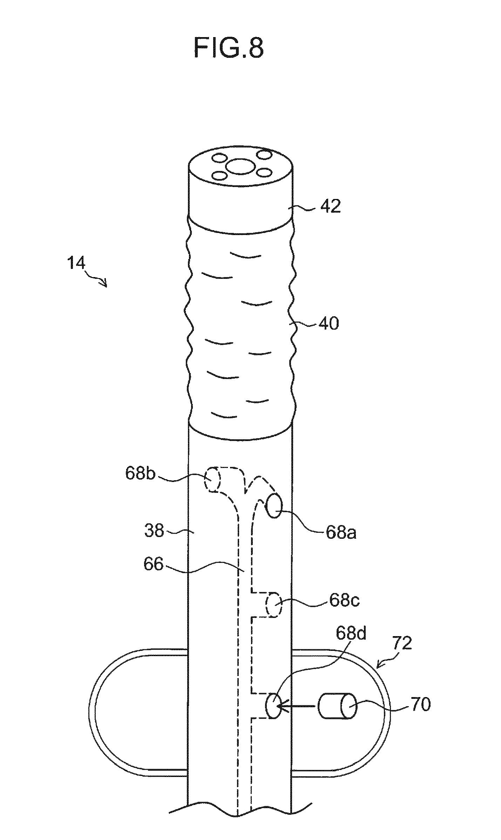

Next, Fig. 8 illustrates a modified example of the third embodiment.

All the plurality of jetting ports 68 are not always used. As illustrated in Fig. 8, it is also possible to avoid using, for example, the jetting port 68d by inserting a stopper plug 70 into the jetting port 68d to thereby prevent the gas from being jetted therefrom. The stopper plug 70 is detachable, and thus can be detached when the jetting port 68d is used.

In addition, as illustrated in Fig. 8, a balloon 72 can be attached to the flexible part 38. For example, in the case where an inflation of the lumen is not enough (insufficient), the stopper plug 70 is detached, and the balloon 72 is attached, whereby the inflation on a forward side can be strengthened.

In addition, it is preferable that a pipe conduit inside of the jetting port 68 have as smoothly curved a shape as possible as illustrated in Fig. 7, in terms of an improvement in cleaning performance inside of the pipe conduit with a brush or other tools, but the pipe conduit may have a shape bent at an acute angle to its axial line as the jetting port 68a illustrated in Fig. 8, or may have a shape bent at a right angle to its axial line as the jetting port 68b illustrated in Fig. 8. The gas can be jetted in a direction farther from the observation window by making the shape of the pipe conduit bent at an acute or right angle to its axial line, and even if bubbles are generated, the field of view of the observation window can be sufficiently secured.

In this way, as in embodiments illustrated in Fig. 7 and Fig. 8, the jetting port is formed on the side surface of the flexible part 38, whereby the jetting port is prevented from being submerged under water even if the leading end part 42 is submerged under water inside of the lumen of the stomach, the large intestine, or other organs. Accordingly, it is possible to suppress bubbles from being generated by the gas jetted from the jetting port, and the field of view of the observation window can be secured.

In addition, as described above, the image-pickup module such as the CCD is placed in the leading end part 42, and hence a restriction on, particularly, the diameter is large in the endoscope leading end part 42. In this regard, because the jetting port is provided in the flexible part 38 located on the base end side of the curving part 40, the gas supply pipe conduit and the jetting port do not need to be placed in the leading end part 42. Accordingly, the jetting port and the like do not affect the diameter of the leading end part 42, thus contributing to a reduction in diameter of the endoscope leading end part 42.

Hereinabove, the endoscope and the endoscope system according to the present invention have been described in detail. It goes without saying that the present invention is not limited to the above-mentioned embodiments, and may be variously improved or modified within a range not departing from the gist of the present invention.

## Claims

1. An endoscope (10) comprising:
a hand operation section (12);
an insertion section (14) including a leading end part (42), a curving part (40), and a flexible part (38) that are placed in the stated order from a leading end side to a base end side on which the hand operation section (12) is placed,
the leading end part (42) having a leading end surface (44) on which an observation window for observing an inside of a subject's body is provided,
the curving part (40) being remotely operated for curving by operating the hand operation section (12) so as to cause the leading end part (42) to face a desired direction,
the flexible part (38) connecting the curving part (40) with the hand operation section (12) and being able to curve in a desired direction along an insertion into the subject's body;
a gas supply channel formed inside of the insertion section (14); and
a gas jetting port (54, 64, 68) that is communicated with the gas supply channel, **characterized in that** the gas jetting port (54, 64, 68) is placed so as to be closer to the base end side than the leading end surface (44) on which the observation window (46) is provided.

2. The endoscope according to claim 1,
wherein the gas jetting port (54) is placed on a stepped surface that is formed by providing a step on part of the leading end surface (44) so as to be lower toward the base end side than the leading end surface (44).

3. The endoscope according to claim 1,
wherein the gas jetting port (64) is formed on a side surface of the leading end part (42).

4. The endoscope according to claim 1,
wherein the gas jetting port (68) is formed so as to be closer to the base end side than the curving part (40).

5. The endoscope according to claim 4,
wherein the gas jetting port (68) is formed on a side surface of the flexible part (38).

6. The endoscope according to claim 5,
wherein a plurality of the gas jetting ports (68a, 68b) are formed in a circumferential direction of the flexible part (38) on the side surface of the flexible part (38).

7. The endoscope according to claim 5,
wherein a plurality of the gas jetting ports (68a, 68c, 68d) are formed in an axial direction of the flexible part (38) on the side surface of the flexible part (38).

8. The endoscope according to any one of claim 3 and claims 5 to 7,
wherein a pipe conduit portion of the gas supply channel (66) communicated with the gas jetting port (68a) is smoothly curved in proximity to the gas jetting port (68a).

9. The endoscope according to any one of claim 3 and claims 5 to 7,
wherein a pipe conduit portion of the gas supply channel (66) communicated with the gas jetting port (68a, 68b) is bent at one of a substantially right angle and an acute angle to an axial direction of the endoscope in proximity to the gas jetting port (68a, 68b).

10. An endoscope system comprising:
the endoscope according to any one of claims 1 to 9; and
a gas supply apparatus (300) that supplies a gas to the gas supply channel of the endoscope.

11. The endoscope system according to claim 10,
wherein the gas supply apparatus (300) continuously supplies the gas.
